# EUROPEAN PATENT SPECIFICATION

(11) **EP 3 482 725 B1**
(45) Date of publication and mention of the grant of the patent: **22.04.2020**
(21) Application number: 18157124.1
(22) Date of filing: 16.02.2018
(51) Int. Cl.: A61F 5/01, A61F 13/06, H05B 6/12

(54) **DEVICE FOR THE TREATMENT OF METATARSOPHALANGEAL PREDISLOCATION SYNDROME**
VORRICHTUNG ZUR BEHANDLUNG DES GROSSZEHENGRUNDGELENK-PRÄDISLOKATIONSSYNDROMS
DISPOSITIF POUR LE TRAITEMENT DU SYNDROME DE PRÉDISLOCATION MÉTATARSOPHALANGIENNE

(30) Priority: 10.11.2017 ES 201731364 U
(43) Date of publication of application: 15.05.2019
(73) Proprietor: Lucas Picazo, David, 03008 Alicante (ES); Monzó Pérez, Francisco, 03600 Elda (Alicante) (ES)
(72) Inventor: Lucas Picazo, David, 03008 Alicante (ES); Monzó Pérez, Francisco, 03600 Elda (Alicante) (ES)
(74) Representative: Ibarra Garcia, Isabel

(56) References cited:
- WO-A1-2016/066867
- US-A- 5 282 782
- US-A1- 2012 136 293
- US-A1- 2012 232 453

## Description

### OBJECT OF THE INVENTION

The present invention relates to a device for the treatment of metatarsophalangeal predislocation syndrome (floating toe), specifically provided for addressing those cases of metatarsalgia caused by metatarsophalangeal predislocation syndrome or other mechanical causes responsible for pain in the sole of the foot (plantar forefoot) that is easy for the patient to put on, highly effective and durable. Document US 2012/232453 A1 represents the closest prior art.

### BACKGROUND OF THE INVENTION

As is known, the painful instability of the lesser toe metatarsophalangeal (MTP) joint, which is most common in the second and third toes, is associated with abnormal load-bearing patterns on the forefoot, such as the progressive subluxation of the metatarsophalangeal joint due to the weakening of or damage to the tissue in stabilizing periarticular structures, such as the intrinsic and extrinsic muscles of the foot, the aponeurotic plantar plate flexor, metatarsophalangeal collateral ligaments, etc. It is also associated with progressive processes with medial/lateral deviation and/or dorsiflexion of the proximal phalange of the metatarsophalangeal joint, where it may even manifest along with the presence of metatarsal plantar hyperkeratosis (calluses/patches of hard skin on the sole) in the region of the second and third metatarsal bone.

A study of the causes that may be responsible for the aforementioned deformations provides the following as possible causes:

### • Hallux valgus

- Hypermobility of the first ray, low degree of rigidity of the medial column of the foot.
- Excessively long second metatarsal bone or *index minus,* the most common.
- Shortening of the gastrocnemius and soleus muscles.
- Inflammatory joint diseases: synovitis, RA, connective tissue disorders that weaken the collateral ligaments.
- Structural deformities such as mallet toe, claw toe, etc.

Clinically speaking, the symptoms of metatarsophalangeal predislocation syndrome include the following:
- Pain in the plantar region before the head and in the dorsal plantar region of the metatarsophalangeal joint involved.
- Pain due to metatarsophalangeal plantar flexion.
- Pain increases with walking or load-bearing activities.
- Pain subsides with rest and by taking NSAIDs.
- "Massage disease": The patient feels alleviated by massaging the region and continues to be in pain while standing.
- Feeling of "walking on a stone."
- Feeling of the toe being out of place. Floating toe.
- Unable to walk barefoot or on hard surfaces.
- Positive Lachman and Kelikian tests.

The differential diagnosis associated with the metatarsophalangeal predislocation syndrome can be the following:
- Stress fracture.
- Claw toe.
- Rheumatoid arthritis, inflammatory arthropathies.
- Freiberg disease.
- MTP synovitis of an arthritic origin.

When metatarsophalangeal predislocation syndrome is detected today, conservative methods are applied, such methods including dressing-type bandages, which are very primitive and must be replaced every two or three days, requiring a very complicated placement that the patient will most likely not be able to reproduce, leading to low effectiveness.

Additionally, the dressing treatments prevent the skin from breathing and are very uncomfortable.

Among the most typical dressings are those referred to as a cravat bandage, while there are other treatments, such as pharmacological treatments, the use of orthopedic insoles with relief elements such as metatarsal pads and/or a horseshoeshaped metatarsal pad, modifying footwear, physiotherapeutic treatment, etc.

Surgery is typically employed when conservative treatment is ineffective and the ultimate purpose of which is to correct the deformity and prevent pain, with restoration of the plate of the joint and with the loosening of the contracted structures, where the scope and the technique to be used depend on the degree of severity of the dislocation.

Based on the foregoing, the applicant of this utility model understands the need that exists for a device to solve the problems set forth in a satisfactory manner.

### DISCLOSURE OF THE INVENTION

The proposed device for the treatment of metatarsophalangeal predislocation syndrome solves the problems set forth above in a fully satisfactory manner based on a simple but effective solution.

To that end, the proposed device is provided to offer an alternative treatment to cravat bandaging and to solve the cases of metatarsalgia brought about by predislocation syndrome or other mechanical causes generating pain in the forefoot.

More specifically, the device of the invention is made up of an elastic tubular body, that is applied in the anterior region of the foot, with said anterior region being tightly housed in the tubular body, with the particularity that a frontal area which should be located in the forefoot, specifically on the proximal phalange of the predislocated toe, emerges from the anterior edge, and from this frontal emergence two cinch-like side flaps are formed that can be folded to go around the dislocated toe and preferably have a different width and/or length in order to adapt to the ergonomics of the foot.

The flap having a smaller width is thereby inserted between the predislocated toe and the toe next to it, preferably the big toe, projecting towards the sole of the foot, i.e., backwards and under the foot, to be fixed on the opposite face of the tubular body, specifically to be fixed to the lower part of the tubular body, while the other flap having a greater width is located on the remaining toes, placed on top of them and laterally folded downwards likewise to be fixed on the lower part of the tubular body located on the sole of the foot.

The fixing of the flaps is performed by fixing means, such as Velcro ® straps or the like.

The proposed configuration offers the following advantages:
- Handling and use that is simpler for the patient.
- Adjacent horseshoe-type metatarsal relief.
- Longer mean usage life.
- Relief and correction of the position of any predislocated or displaced toe. It should be pointed out that the indication of said device covers a wide range of treatment options for metatarsal pain. It is indicated as a treatment of choice in acute inflammatory processes (post-surgical immobilizations, metatarsophalangeal synovitis, bone fractures in the toes, etc.) and/or painful chronic processes at the metatarsal level.
- Breathable and washable material (unlike the tape for the dressing) without skin irritation.
- It is a conservative treatment option that is complementary to the use of an orthopedic insole.

### DESCRIPTION OF THE DRAWINGS

To complement the description that will be made below and for the purpose of aiding to better understand the features of the invention according to a preferred practical embodiment thereof, a set of drawings is attached as an integral part of said description in which the following is depicted for an illustrative and non-limiting purpose:
Figure 1 shows a general perspective view general from the lower part of a device for the treatment of metatarsophalangeal predislocation syndrome according to the object of the present invention.
Figure 2 shows a perspective view of the application of the device in an initial phase of implementing same.
Figure 3 shows a perspective view similar to the view in the preceding drawing, corresponding to the final assembly phase.
Figure 4 shows a bottom perspective view of the initial phase depicted in Figure 2.
Figure 5 shows a perspective view similar to the view in the preceding drawing, in a later phase, in which the flaps are taken along the sides of the predislocated toe.
Figure 6 shows a perspective view similar to the view in the preceding drawing, in a final assembly phase.

### PREFERRED EMBODIMENT OF THE INVENTION

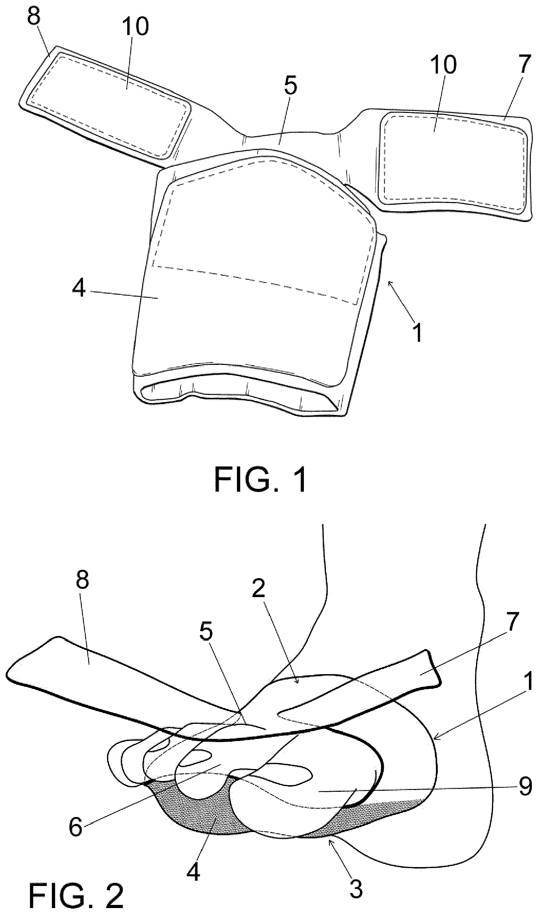

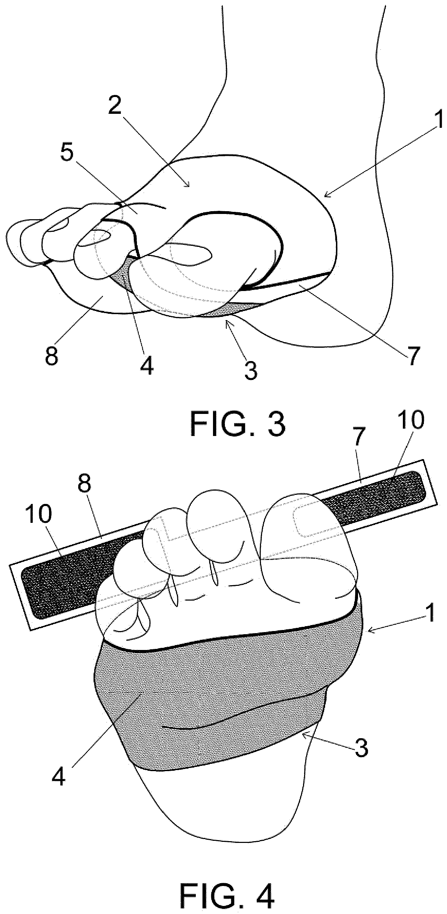

In view of Figures 1 and 3, it can be seen how the device for the treatment of metatarsophalangeal predislocation syndrome of the invention is made up of a tubular body (1), made of an elastic breathable material, in which the tubular body is arranged on the anterior region of the instep of the foot, arranged such that two faces or surfaces provided with distinctive technical elements are distinguished.

In view of Figure 2, the face of the tubular body (1) arranged in the upper part (2) of the foot can be seen, having a frontal area (5) from which there emerge two flaps or cinches, while the face of the tubular body arranged in the lower part of the foot has a lower part (3) provided with a covering of an adhesive material (4), such as Velcro ® or the like, the function of which will be explained below.

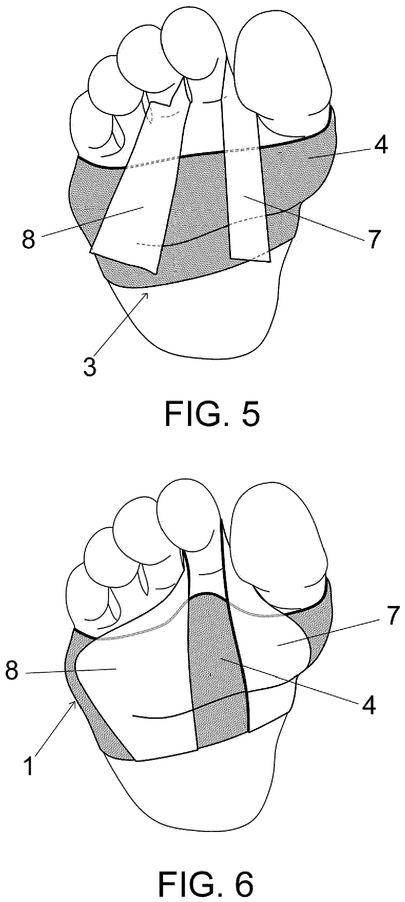

Preferably, as seen in Figures 2, 4, 5 and 6, the frontal area (5) emerging from the anterior edge and in correspondence with the upper part (2) of the tubular body (1) of the device is located on the first phalange of the dislocated toe (6), having a length in accordance with the latter, such that there emerge from said frontal area (5) the two aforementioned side flaps (7) and (8), the former having a smaller width and being intended for being taken between the toe immediately to the left of the dislocated toe, in this case the big toe (9), and said dislocated toe (6) to be fixed on the lower adhesive part (4) of the tubular body (1) as a result of the flaps (7) and (8) being provided on the inner face thereof with respective adhesive straps (10), such as Velcro ® straps, for example, the other flap (8) being fixed in a similar manner, as shown in Figures 5 and 6.

Additionally, it is possible to include two Velcro ® straps arranged on the face of the tubular body that is located on the sole of the foot, which embodiment is not depicted in the described figures.

The flaps (7) and (8) will be fixed on these additional straps in an aligned manner so as to provide a raised central region on the foot providing greater relief and comfort that will in certain cases provide overall alleviation for the patient.

## Claims

1. A device for the treatment of metatarsophalangeal predislocation syndrome, that is made up of an elastic tubular body (1), made of a breathable material, which is adapted in shape and dimensions to the anterior region of the instep of the foot, said tubular body having, in correspondence with the anterior edge of its face (2), arranged in the upper part of the foot in use, a frontal area (5) having a length in accordance with the phalange of the dislocated toe (6) in use, where it is from this frontal area (5) that two side flaps (7) and (8) project, which side flaps (7) and (8) can be folded, **characterized in that** said side flaps (7) and (8) have fixing means (10) on the inner face thereof for fixing them on the face arranged on the sole of the foot in use, going around the dislocated toe (6).

2. The device for the treatment of metatarsophalangeal predislocation syndrome according to claim 1, **characterized in that** the side flaps (7) and (8) have different lengths.

3. The device for the treatment of metatarsophalangeal predislocation syndrome according to claim 1, **characterized in that** the side flaps have different widths.

4. The device for the treatment of metatarsophalangeal predislocation syndrome according to claim 1, **characterized in that** the fixing means are Velcro ® straps or the like.

## Patentansprüche

1. Vorrichtung zur Behandlung des Großzehengrundgelenk-Prädislokationssyndroms, welche aus einem elastischen rohrförmigen Körper (1), hergestellt aus einem luftdurchlässigen Material, besteht, welcher in Form und Abmessung an den Vorderbereich des Spanns des Fußes angepasst ist, wobei der genannte rohrförmige Körper, in Übereinstimmung mit dem Vorderrand dessen Fläche (2), im oberen Teil des Fußes in Verwendung angeordnet, eine Stirnzone (5) aufweist, welche eine Länge gemäß dem Zehenglied des dislozierten Zehs (6) in Verwendung aufweist, wobei zwei Seitenlaschen (7) und (8) aus dieser Stirnzone (5) herausragen, welche Seitenlaschen (7) und (8) gefaltet werden können, **dadurch gekennzeichnet, dass** die genannten Seitenlaschen (7) und (8) Befestigungsmittel (10) auf der Innenfläche derselben aufweisen, zur Befestigung derselben auf der Fläche, welche auf der Sohle des Fußes in Verwendung angeordnet ist, welche um den dislozierten Zeh (6) herum gehen.

2. Vorrichtung zur Behandlung des Großzehengrundgelenk-Prädislokationssyndroms nach Anspruch 1, **dadurch gekennzeichnet, dass** die Seitenlaschen (7) und (8) unterschiedliche Längen aufweisen.

3. Vorrichtung zur Behandlung des Großzehengrundgelenk-Prädislokationssyndroms nach Anspruch 1, **dadurch gekennzeichnet, dass** die Seitenlaschen unterschiedliche Breiten aufweisen.

4. Vorrichtung zur Behandlung des Großzehengrundgelenk-Prädislokationssyndroms nach Anspruch 1, **dadurch gekennzeichnet, dass** die Befestigungsmittel Velcro®-Bänder oder ähnliche sind.

## Revendications

1. Dispositif pour le traitement du syndrome de prédislocation métatarsophalangienne, qui est composé d'un corps tubulaire élastique (1) en matière respirante qui s'adapte à la forme et aux dimensions de la zone antérieure du cou de pied, ledit corps tubulaire ayant, en correspondance avec le bord antérieur de sa face (2), aménagé dans la partie supérieure du pied utilisé, un secteur avant (5) ayant une longueur appropriée à la phalange du doigt disloqué (6) utilisé, lequel secteur avant (5) étant celui d'où sortent deux rabats latéraux (7) et (8), lesdits rabats latéraux (7) et (8) peuvent être rabattus, **caractérisé en ce que** lesdits rabats latéraux comportent des moyens de fixation (10) sur leur face interne en vue de leur fixation sur la face disposée sur la plante du pied utilisé, entourant le doigt disloqué (6).

2. Dispositif pour le traitement du syndrome de prédislocation métatarsophalangienne selon la revendication 1, **caractérisé en ce que** les rabats latéraux (7) et (8) ont des longueurs différentes.

3. Dispositif pour le traitement du syndrome de prédislocation métatarsophalangienne selon la revendication 1, **caractérisé en ce que** les rabats latéraux ont des largeurs différentes.

4. Dispositif pour le traitement du syndrome de prédislocation métatarsophalangienne selon la revendication 1, **caractérisé en ce que** les moyens de fixation sont des bandes de Velcro@ ou analogues.
